# EUROPEAN PATENT SPECIFICATION

(11) **EP 1 151 092 B1**
(45) Date of publication and mention of the grant of the patent: **31.12.2008**
(21) Application number: 00908604.2
(22) Date of filing: 11.02.2000
(51) Int. Cl.: C12N 15/11, G01N 33/68, A61P 9/10

(54) **INHIBITING FORMATION OF ATHEROSCLEROTIC LESIONS WITH AFABP ANTISENS NUCLEIC ACIDS**
HEMMUNG DER BILDUNG VON ATHEROSKLEROTISCHEN WUNDEN MITTELS AFABP ANTISENS NUKLEINSÄUREN
INHIBITION DE LA FORMATION DES LESIONS ATHEROSCLEREUSES AVEC DES ACIDES NUCLÉIQUES ANTISENS D'AFABP

(30) Priority: 12.02.1999 US 119880 P
(43) Date of publication of application: 07.11.2001
(73) Proprietor: President and Fellows of Harvard College, Cambridge, MA 02138 (US)
(72) Inventor: HABER, Edgar, (US); LEE, Mu-En, Newton, MA 02159 (US); PERRELLA, Mark, A., Brookline, MA 02146 (US); HOTAMISLIGIL, Gokhan, S., Cambridge, MA 02142 (US)
(74) Representative: Crump, Julian Richard John
(86) International application number: PCT/US2000/003560
(87) International publication number: WO 2000/047734

(56) References cited:
- WO-A-00/15230
- WO-A-92/06104
- WO-A-98/45440
- RICHIERI G V ET AL: "EQUILIBRIUM CONSTANTS FOR THE BINDING OF FATTY ACIDS WITH FATTY ACID-BINDING PROTEINS FROM ADIPOCYTE, INTESTINE, HEART, AND LIVER MEASURED WITH THE FLUORESCENT PROBE ADIFAB" JOURNAL OF BIOLOGICAL CHEMISTRY, vol. 269, no. 39, 30 September 1994 (1994-09-30), pages 23918-23930, XP000645401 ISSN: 0021-9258 cited in the application
- LYLE ROBERT E ET AL: "Antisense oligonucleotides to differentiation-specific element binding protein (DSEB) mRNA inhibit adipocyte differentiation." BIOCHEMICAL AND BIOPHYSICAL RESEARCH COMMUNICATIONS, vol. 228, no. 3, 1996, pages 709-715, XP002139363 ISSN: 0006-291X
- HOTAMISLIGIL GOKHAN S ET AL: "Uncoupling of obesity from insulin resistance through a targeted mutation in aP2, the adipocyte fatty acid binding protein." SCIENCE, vol. 274, no. 5291, 1996, pages 1377-1379, XP002139364 ISSN: 0036-8075 cited in the application
- HORVAI A. ET AL.: "Scavenger receptor A gene regulatory elements target gene expression to macrophages and to foam cells of atherosclerotic lesions." PROC NATL ACAD SCI U S A 1995 JUN 6;92(12):5391-5, XP002139365 cited in the application
- PELTON PATRICIA D ET AL: "PPARgamma activation induces the expression of the adipocyte fatty acid binding protein gene in human monocytes." BIOCHEMICAL AND BIOPHYSICAL RESEARCH COMMUNICATIONS, vol. 261, no. 2, 2 August 1999 (1999-08-02), pages 456-458, XP002139366 ISSN: 0006-291X
- WOLFRUM C ET AL: "Variation of liver-type fatty acid binding protein content in the human hepatoma cell line HepG2 by peroxisome proliferators and antisense RNA affects the rate of fatty acid uptake." BIOCHIMICA ET BIOPHYSICA ACTA, (1999 FEB 25) 1437 (2) 194-201., XP000907541

## Description

### TECHNICAL FIELD

This invention relates to treatment of cardiovascular diseases.

### BACKGROUND

Atherosclerosis is a slow, progressive disease which may begin in childhood. In some individuals, the disease progresses rapidly in the third decade in life, while in others, the disease is not evident until the fifth or sixth decade of life. The disease is characterized by plaque or atherosclerotic lesion formation which is thought to begin when the innermost layer of the artery becomes damaged. Lipids, cholesterol, fibrin, platelets, cellular debris and calcium are deposited at the damaged site in the artery wall to form a lesion. The disease affects medium-sized and large arteries and is characterized by lesion formation which partially or totally blocks the flow of blood through an artery.

### SUMMARY

The invention is based on the discovery that decreasing adipocyte fatty acid binding protein (AFABP or aP2) expression inhibits atherosclerotic lesion formation. Accordingly, the invention provides means for inhibiting formation of atherosclerotic lesions by administering to a mammal, e.g., a human patient who has been identified as suffering from or at risk of developing atherosclerosis, a compound that reduces expression of AFABP. The compound inhibits transcription of AFABP. More preferably, the compound inhibits expression of AFABP in macrophages but not in adipocytes. Alternatively, the compound inhibits AFABP expression in both macrophages and adipocytes.

The compound inhibits translation of AFABP mRNA into an AFABP gene product, i.e., an antisense nucleic acid. An antisense nucleic acid molecule contains at least 10 nucleotides the sequence of which is complementary to an mRNA encoding an AFABP polypeptide. Preferably, the compound, e.g., an antisense oligonucleotide or antisense RNA produced from an antisense template, inhibits AFABP expression by inhibiting translation of AFABP mRNA. For example, antisense therapy is carried out by administering a single stranded nucleic acid complementary at least a portion of AFABP mRNA. In another example, the antisense nucleic acid is a DNA operatively linked to a macrophage-specific promoter, and the transcription of DNA yields nucleic acid product which is complementary to an mRNA encoding an AFABP polypeptide. The compound is administered systemically or locally, e.g., it is introduced into an artery of the mammal or administered directly to the site of an atherosclerotic lesion using a medical device such as a catheter or vascular stent.

The invention also includes a method of inhibiting differentiation of a macrophage into a foam cell by contacting a monocyte or macrophage with an inhibitor of AFABP expression or activity.

Compounds that inhibit binding of AFABP to a fatty acid, e.g., oleic acid or retinoic acid, are identified in vitro by contacting an AFABP polypeptide with a fatty acid in the presence of a candidate compound and determining the level of AFABP binding to the fatty acid. A decrease in the level of binding in the presence of the candidate compound, compared to the level of binding in the absence of the candidate compound is an indication of the that the candidate compound inhibits AFABP/fatty acid binding. Alternatively, an in vitro screening method to identify such compounds is carried out by providing an AFABP polypeptide with a fatty acid bound in a complex, contacting the complex with a candidate compound, and determining whether the candidate compound decreases the binding of AFABP to a fatty acid in the complex as an indication of the ability of the candidate compound to inhibit AFABP binding.

The invention also includes in vitro methods of screening for compounds that inhibit atherosclerotic lesion formation, e.g., by inhibiting AFABP expression in a cell or inhibiting AFABP activity. A method for identifying a compound which inhibits AFABP expression in a macrophage, that either naturally expresses AFABP or has been genetically engineered to do so, is carried out by providing a cell that expresses AFABP, culturing the cell in the presence of a candidate compound, determining the level of expression of a AFABP, and comparing the level detected in cells cultured in the presence and absence of the candidate compound. For example, a decrease in the level of expression in the presence of the candidate compound compared to the level of expression in the absence of the candidate compound indicates that the candidate compound inhibits AFABP expression, and as a result, inhibits development of atherosclerosis. To determine whether the compound preferentially inhibits expression in macrophages compared to adipocytes, both cell types are contacted with the same candidate compound in parallel, and the level of expression of AFABP in macrophages and adipocytes is compared. A decrease in expression in macrophages compared to that in adipocytes indicates that the compound preferentially decreases AFABP expression in macrophages. Similarly, A decrease in expression in adipocytes compared to that in macrophages indicates that the compound preferentially decreases AFABP expression in adipocytes.

Compound which inhibit AFABP activity by reducing the binding of AFABP to an intracellular ligand in a macrophage are identified in vitro by providing a macrophage that expresses AFABP, culturing the macrophage in the presence of a candidate compound, and determining the level of AFABP binding to its ligand in the macrophage. A decrease in the level of binding in the presence of the compound compared to the level of binding in the absence of the compound is an indication that the compound inhibits AFABP binding to an intracellular ligand, e.g., a long chain fatty acid, thereby inhibiting atherosclerotic lesion formation.

Cholesteryl esters (esterified cholesterol) accumulate in macrophages present in atherosclerotic lesions or plaques. A method to identify a compound which inhibits such cholesterol loading in macrophages is carried out by culturing a macrophage expressing AFABP in the presence of a candidate compound, measuring the level of cholesterol in the macrophage in the presence of the compound and comparing the level to the level in an AFABP-expressing macrophage cultured in the absence of the compound. A reduction in the level of cholesterol in macrophage cultured in the presence of the compound compared to the level in a macrophage cultured in the absence of the compound indicates that the compound inhibits cholesterol-loading in macrophages and inhibits formation of atherosclerotic lesions.

Other features and advantages of the invention will be apparent from the following description of the preferred embodiments thereof, and from the claims.

### DESCRIPTION OF DRAWINGS

Fig. 1 is a bar graph showing the effect of AFABP deficiency on body weight of apoE deletion mice.
Fig. 2 is a bar graph showing the effect of AFABP deficiency on serum cholesterol in apoE deletion mice.

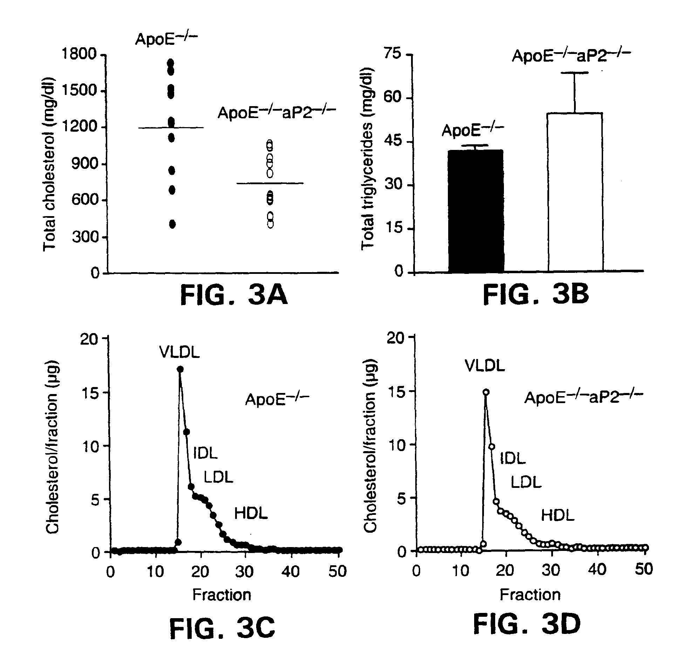

Figs 3A-D are diagrammatic representations of data from a lipoprotein analysis in ApoE-/- and ApoE-/- AFABP-/- mice. Fig. 3A is a diagram showing serum levels of total cholesterol (horizontal lines show mean; black ovals indicate ApoE-/-; white ovals indicate ApoE-/- AFABP-/-) Fig. 3B is a bar graph showing triglycerides (mean ±SE; black bars indicate ApoE-/-; white bars indicate ApoE-/- AFABP-/-. Fig. 3C is a line graph showing the lipoprotein fractions of serum from ApoE-/- mice (black circles), and Fig. 3D is a line graph showing the lipoprotein fractions of serum from ApoE-/- mice (white circles). The mice were also analyzed by FPLC. VLDL = very low density lipoproteins; IDL = intermediate density lipoproteins; LDL = low density lipoproteins; and HDL = high density lipoproteins.
Fig. 4A is a diagram of aortic arch and arterial branches analyzed for atherosclerotic lesions, including the brachiocephalic (Br, proximal and distal portions), right subclavian (RSC), right common carotid (RCC), left subclavian (LSC), and left common carotid (LCC) arteries.
Fig. 4B is a bar graph showing the mean cross-sectional area (mean ± SE) of atherosclerotic lesions from ApoE -/- mice (black bars) and ApoE-/- AFABP-/- mice (white bars).

### DETAILED DESCRIPTION

Hypercholesterolemia is a major risk factor in coronary heart disease, e.g., atherosclerosis. The data described herein uncouples the link between atherosclerosis and hypercholesterolemia. Inhibiting AFABP expression or activity reduces the development of atherosclerotic lesions despite a high level of serum cholesterol.

### Effect of AFABP deficiency on body weight, serum cholesterol, and neointima formation in arteries of apoE deficient mice

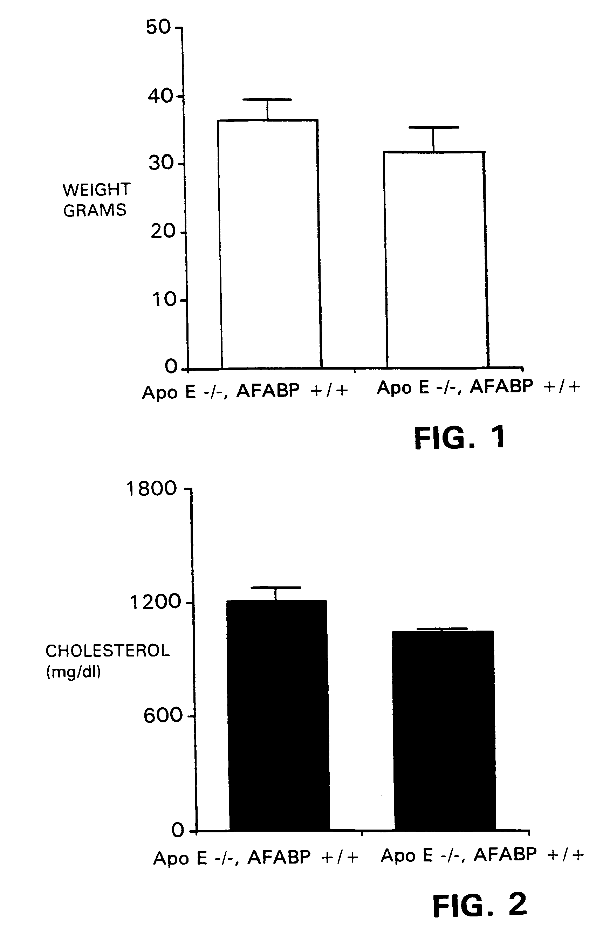

Mice with a null mutation in the genes for apoE (apoE -/-) or both apoE and AFABP (apoE -/-, AFABP -/-) were used. Mice from both groups were weaned at 4 weeks of age and then place on a Western-type diet (Tekland Adjusted Calories Western-type diet, Harlan-Tekland, Madison, WI) which contained 21 % fat by weight. The Western-type diet and water were provided ad libitum for a 12 week period. Body weight (Fig. 1) and total serum cholesterol (Fig. 2) were assessed in both groups (n=3) at the end of 12 weeks on Western-type diet.

After 12 weeks on a Western-type diet, apoE -/- mice and apoE -/-, AFABP -/mice were anesthetized with sodium pentobarbital. The vasculature of the mice was perfused with phosphate buffered saline (PBS). The arch of the aorta and the right brachiocephalic artery (including the more distal right common carotid and subclavian arteries) were dissected and fixed in methyl Carnoy's solution at 4E C prior to being embedded in paraffin. The vascular tissue was then subjected to histological analysis.

The riboprobe used for detection of AFABP expression in situ hybridization experiments was prepared from a plasmid containing mouse AFABP cDNA. The vector was digested with Sal I to remove the 3' end of the cDNA and religated. The resulting plasmid was verified by sequencing. The plasmid was then linearized with Sal I to generate a template for sense riboprobe synthesis with SP6 RNA polymerase. The plasmid was also linearized with Pst I to generate a template for anti□sense riboprobe synthesis with T7 RNA polymerase. An antisense nucleic acid for therapeutic purposes is generated using the same templates and standard methods. Alternatively, the antisense nucleic acid is chemically synthesized. The sequence of the 5' Sal I fragment (sense) of the mouse AFABP cDNA used for riboprobe was: cctttctcacctggaagacagctcctcctcgaaggtttacaaaatgtgtgatgcctttgtgggaacctggaagcttgtct ccagtgaaaacttcgatgattacatgaaagaagtgggagtgggctttgccacaaggaaagtggcaggcatggccaa gcccaacatgatcatcagcgtaaatggggatttggtcaccatccggtcagagagtacttttaaaaacaccgagatttc cttcaaactgggcgtggaattcgatgaaatcaccgcagacgacaggaaggtgaagagcatcataaccctagatggc ggggccctggtgcaggtgcagaagtgggatggaaagtcgac (SEQ ID NO:6)

Sequential tissue sections (5 microns in thickness and approximately 60 microns between sections) were stained for elastin. Tissue sections were analyzed throughout each lesion (e.g., sections were taken starting at the beginning of the right brachiocephalic artery from the arch of the aorta (covering a maximal distance of 600 microns). Photomicrographs of carotid artery tissue sections were taken to evaluate the effect of AFABP deficiency on neointima formation in brachiocephalic arteries of apoE deficient mice. The proximal and middle portions of the lesions in apoE -/- mice and corresponding tissue sections from apoE -/-, AFABP -/- mice were examined. The data indicate that complex atherosclerotic lesion formation in apoE -/- is inhibited in the absence of AFABP. ApoE -/- mice maintained on the Western-type diet have cholesterol levels of greater than 1000. These mice also develop severe atherosclerosis, e.g., the carotid artery typically becomes 65-95% occluded. In contrast, in double mutant apoE -/-, AFABP -/- mice, few or no atherosclerotic lesions were detected. Double knockout mice generally weighed less than apoE -/- single knockout mice. For example, double knockout mice typically weighed 15-20% less than single apoE -/- mice. The level of cholesterol in double knockout mice was significantly less than that in apoE single knockout mice (1046 ± 17 mg/dl in double knockout mice compared to 1201 ± 73 mg/dl in single knockout mice). These data indicate that AFABP-deficient mice are resistant to hypercholesterolemia.

Macrophages of apoE -/-, AFABP -/- mice were found to be less able to load cholesterol. A 60% reduction in cholesterol loading was observed in the double knockout mice compared to the single knockout (apoE -/-) mice. These data indicate that inhibition of AFABP expression or activity in macrophages reduces or prevents the formation of atherosclerotic lesions in a mammal even in the presence of high serum cholesterol levels.

### Assessment of AFABP mRNA from human monocytes and mouse peritoneal macrophages

The data described herein indicates that AFABP is expressed, not only in adipocytes, but also in macrophages, a cell type which participates in the development of atherosclerotic lesions.

To study AFABP expression, mRNA was prepared from mouse peritoneal macrophages and human monocytes differentiated into macrophages. Northern blot assays were carried out. Mouse peritoneal macrophages were harvested 5 days after intraperitoneal administration of thioglycolate. The macrophages from wild-type mice (AFABP +/+) or AFABP -/- mice were plated in standard media and under standard sterile cell culture conditions. mRNA was extracted at 0, 1, 2, 3, and 4 days after plating. As a positive control, mRNA was also extracted from adipocytes and pre-adipocytes. To study AFABP expression in human cells, human peripheral blood monocytes were isolated by standard Ficoll-Paque centrifugation from the buffy coats of samples obtained from a blood bank. The cells were initially plated for 1 hour, then non-adherent cells were eliminated by washing three times. Adherent cells were approximately 90% monocytes. mRNA was extracted from the cells at the 0, 1, 3 and 5 days after plating. Northern blot analyses were performed according to methods well known in the art using 10 µg of total RNA per lane. Blots were hybridized using a radiolabeled cDNA probe from mouse AFABP. These data indicate that AFABP is expressed in the monocyte/macrophage cell type in addition to adipocytes. The data also indicate that little or no AFABP is expressed by circulating monocytes (0-1 days after plating). Upon differentiation of the monocytes into macrophages, AFABP is expressed at a high level.

### Methods of inhibiting formation of atherosclerotic lesions

The marked decrease in the incidence of atherosclerotic lesions in apoE -/-,

AFABP-/- double knockout mice indicates that AFABP contributes to atherosclerotic lesion formation. Inhibition of lesion formation is achieved by contacting vascular cells with a compound that inhibits AFABP transcription and/or activity.

Nucleic acids complementary to all or part of the AFABP coding sequence. For example, the nucleic acid is at least 10 nucleotides in length (more preferably at least 20, 30, 40, 50 nucleotides in length) which is complementary at least a 10 nucleotide stretch of the murine AFABP cDNA (Table 1, GenBank Accession # K02109; Bernlohr et al., 1984, Proc. Natl. Acad. Sci. U.S.A. 81:5468-5472) or the human AFABP cDNA (Table 2, GenBank Accession # J02874; Baxa et al., 1989, Biochemistry 28:8683□8690) is used in antisense therapy to inhibit expression of AFABP.

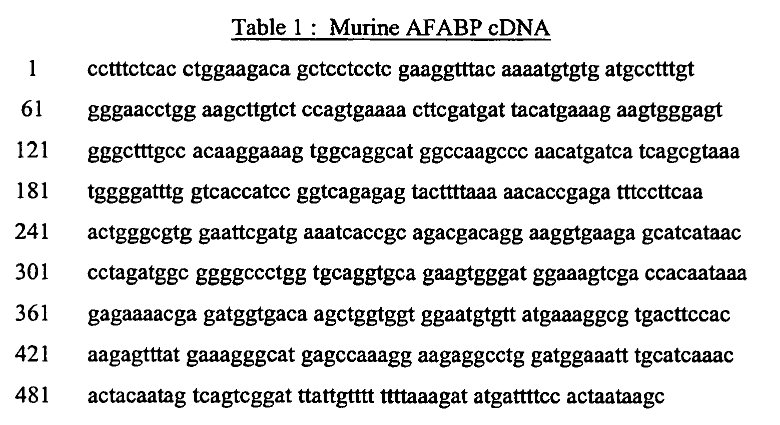

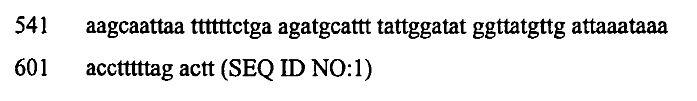

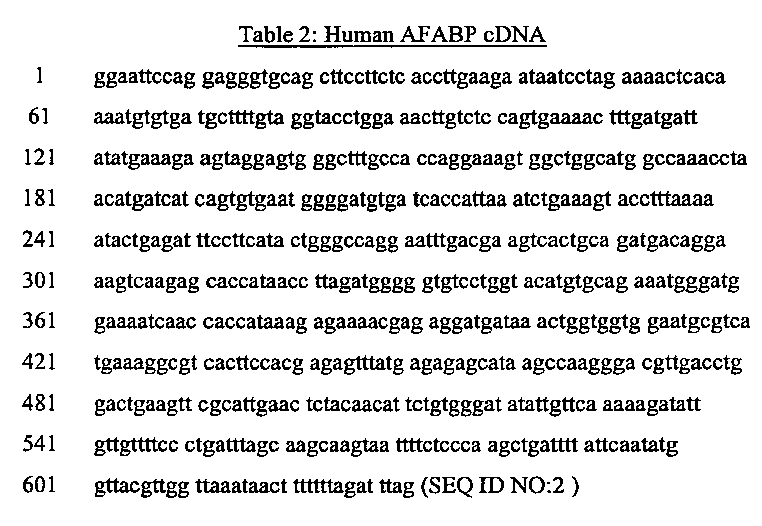

For example, the antisense nucleic acid to be administered has the sequence of the complement of SEQ ID NO:6.

Antisense treatment is carried out by administering to a mammal such as a human patient, DNA containing a promoter, e.g., a macrophage-specific promoter, operably linked to a DNA sequence (an antisense template), which is transcribed into an antisense RNA. For example, the promoter of the scavenger receptor A gene (Horvai et al., 1995, Proc Natl Acad Sci USA 92:5391-5) is operably linked to an antisense template or DNA encoding an AFABP inhibitory peptide to target expression to macrophages and to foam cells of atherosclerotic lesions. Alternatively, antisense oligonucleotides are introduced directly into target cells such as monocytes or macrophages. The antisense oligonucleotide may be a short nucleotide sequence (generally at least 10, preferably at least 14, more preferably at least 20 (e.g., at least 30), and up to 100 or more nucleotides) formulated to be complementary to a portion, e.g., the coding sequence, or all of AFABP mRNA. Standard methods relating to antisense technology have been described (see, e.g., Melani et al., 1991, Cancer Res. 51:2897-2901). Following transcription of a DNA sequence into an antisense RNA, the antisense RNA binds to its target nucleic acid molecule, such as an mRNA molecule, thereby inhibiting expression of the target nucleic acid molecule. For example, an antisense sequence complementary to a portion or all of AFABP mRNA is used to inhibit the expression of AFABP to reduce macrophage-mediated atherosclerotic lesion formation Oligonucleotides complementary to various sequences of AFABP mRNA can readily be tested in vitro for their ability to decrease production of AFABP, using assays described herein. Promising oligonucleotides are tested in vivo in rats or mice, e.g., apoE knockout mice fed a Western diet, to evaluate inhibition of atherosclerosis.

Suitable vectors are known in the art. Preferred vectors are viral vectors, including those derived from replication-defective hepatitis viruses (e.g., HBV and HCV), retroviruses (see, e.g., WO 89/07136; Rosenberg et al., 1990, N. Eng. J. Med. 323(9):570-578), adenovirus (see, e.g., Morsey et al., 1993, J. Cell. Biochem., Supp. 17E,), adeno-associated virus (Kotin et al., 1990, Proc. Natl. Acad. Sci. USA 87:2211-2215,), replication defective herpes simplex viruses (HSV; Lu et al., 1992, Abstract, page 66, Abstracts of the Meeting on Gene Therapy, Sept. 22-26, Cold Spring Harbor Laboratory, Cold Spring Harbor, New York), and any modified versions of these vectors. The invention may utilize any other delivery system which accomplishes in vivo transfer of nucleic acids into eukaryotic cells. For example, the nucleic acids may be packaged into liposomes, receptor-mediated delivery systems, non-viral nucleic acid-based vectors, erythrocyte ghosts, or microspheres (e.g., microparticles; see, e.g., U.S. Patent No. 4,789,734; U.S. Patent No. 4,925,673; U.S. Patent No. 3,625,214; Gregoriadis, 1979, Drug Carriers in Biology and Medicine, pp. 287-341 (Academic Press,). Alternatively, naked DNA may be administered.

Antisense oligonucleotides may consist of DNA, RNA, or any modifications or combinations thereof. As an example of the modifications that the oligonucleotides may contain, inter-nucleotide linkages other than phosphodiester bonds, such as phosphorothioate, methylphosphonate, methylphosphodiester, phosphorodithioate, phosphoramidate, phosphotriester, or phosphate ester linkages (Uhlman et al., 1990, Chem. Rev. 90(4):544-584; Anticancer Research, 1990, 10:1169) may be present in the oligonucleotides, resulting in their increased stability. Oligonucleotide stability is increased by incorporating 3'-deoxythymidine or 2'-substituted nucleotides (substituted with, e.g., alkyl groups) into the oligonucleotides during synthesis, by providing the oligonucleotides as phenylisourea derivatives, or by having other molecules, such as aminoacridine or poly-lysine, linked to the 3' ends of the oligonucleotides. Modifications of the RNA and/or DNA nucleotides may be present throughout the oligonucleotide, or in selected regions of the oligonucleotide, e.g., in the 5' and/or 3' ends. The antisense oligonucleotides are modified so as to increase their ability to penetrate the target tissue by, e.g., coupling the oligonucleotides to lipophilic compounds. Antisense oligonucleotides based on the AFABP nucleotide sequence are generated by any method known in the art, including standard chemical synthesis, ligation of constituent oligonucleotides, and transcription of DNA complementary to the all or part of the AFABP coding sequence.

Prior to the invention, it was thought that AFABP expression was limited to adipocytes. The data described herein indicate that macrophages and macrophage-derived foam cells in atherosclerotic lesions also express this protein. AFABP was found to be expressed in macrophages and macrophage-derived foam cells associated with atherosclerotic lesions (but not circulating monocytes). These cells are, therefore, the preferred cellular targets for antisense therapy. Targeting of antisense oligonucleotides to macrophages is achieved, for example, by coupling the oligonucleotides to ligands of macrophage cell surface proteins, e.g., Fc receptors, receptors for complement proteins, or polysaccharide receptors such as the mannose receptor, the temporal expression of which parallels the expression of AFABP (i.e., the protein is expressed when monocytes mature into macrophages). Similarly, oligonucleotides may be targeted to macrophages by being conjugated to monoclonal antibodies that specifically bind to cell surface proteins, e.g., ICAM and LFA-3.

Methods for therapeutically administering antisense oligonucleotides are known in the art, e.g., as described in the following review articles: Le Doan et al., Bull. Cancer 76:849-852, 1989; Dolnick, Biochem. Pharmacol. 40:671-675, 1990; Crooke, Annu. Rev. Pharmacol. Toxicol. 32, 329-376, 1992. Antisense nucleic acids may be used alone or combined with one or more materials, including other antisense oligonucleotides or recombinant vectors, materials that increase the biological stability of the oligonucleotides or the recombinant vectors, or materials that increase the ability of the therapeutic compositions to penetrate vascular smooth muscle cells selectively.

Instead of inhibiting AFABP transcription and/or translation, the activity of AFABP may be inhibited to treat atherosclerosis. For example, an antibody which binds to AFABP is administered or intracellularly expressed to reduce binding to its intracellular ligand. For administration to human patients, antibodies, e.g., AFABP- specific monoclonal antibodies, are humanized by methods known in the art. Antibodies with a desired binding specificity can be commercially humanized (Scotgene, Scotland; Oxford Molecular, Palo Alto, CA).

Anti-AFABP antibodies are known in the art (e.g., AFABP-specific rabbit polyclonal antisera; Hotamisligil et al., 1996, Science 274:1377-1379) are obtained using techniques well known in the art. Such antibodies are polyclonal or monoclonal. Polyclonal antibodies are generated, e.g., by the methods described in Ghose et al., Methods in Enzymology, Vol. 93, 326-327, 1983. An AFABP polypeptide, or an antigenic fragment thereof, is used as an immunogen to stimulate the production of AFABP-reactive polyclonal antibodies in the antisera of animals such as rabbits, goats, sheep, and rodents. For example, the entire human or murine AFABP is used as an immunogen. Alternatively, an antigenic fragment, an AFABP peptide with the amino acid sequence DKLVVECVMKGVT (SEQ ID NO:3) is used as an immunogen. This peptide is a useful immunogen for the generation of polyclonal antisera or a monoclonal antibody because it is divergent from most of the other fatty acid binding proteins and is conserved in mice and humans.

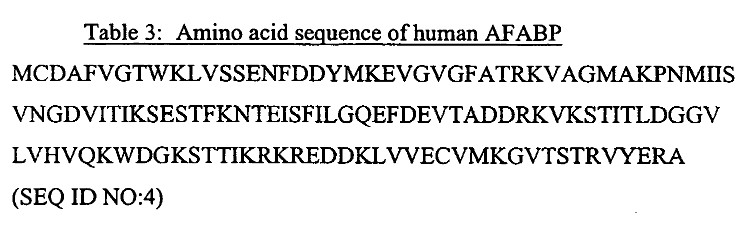

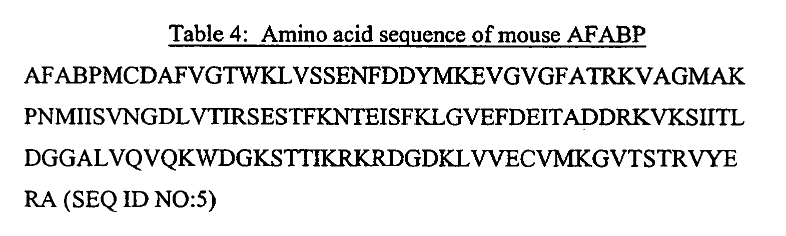

Monoclonal antibodies are obtained using standard techniques, e.g., those described by Milstein and Kohler in Nature, 256:495-97, 1975, or as modified by Gerhard, Monoclonal Antibodies, Plenum Press, 1980, pages 370-371. Hybridomas are screened to identify those producing antibodies that are specific for an AFABP polypeptide. Preferably, the antibody has an affinity of at least about 10⁸ liters/mole and more preferably, an affinity of at least about 10⁹ liters/mole. Following identification of a hybridoma producing a suitable monoclonal antibody, DNA encoding the antibody is cloned. DNA encoding a single chain AFABP-specific antibody in which heavy and light chain variable domains (separated by a flexible linker peptide such as Gly,-Ser3 (SEQ ID NO:7) is cloned into an expression vector using known methods (e.g., Marasco et al., 1993, Proc. Natl. Acad. Sci. USA 90:7889-7893 and Marasco et al., 1997, Gene Therapy 4:11-15). Such constructs are introduced into cells, e.g., using gene therapy techniques described herein, for intracellular production of the antibodies. Intracellular antibodies, i.e., intrabodies, are used to inhibit binding of endogenous AFABP to its intracelluar ligand, which in turn, decreases the activity of AFABP and atherosclerotic lesion formation.

### Administration of therapeutic compositions

Mammals suffering from atherosclerosis are identified using techniques well known in the art (e.g., angiography, electrocardiography as well as physiological and metabolic tests). Individuals at risk of developing atherosclerosis are also treated using the methods described herein. Risk factors include increasing age, male sex, heredity (including race, e.g., African Americans have more severe hypertension and a concomitant greater risk of developing atherosclerosis than whites), cigarette and tobacco smoke, high blood cholesterol levels, high blood pressure, physical inactivity, obesity, stress, and/or diabetes mellitus.

Therapeutic compositions, i.e., nucleic acid-based inhibitors inhibitors of AFABP expression are administered in pharmaceutically acceptable carriers (e.g., physiological saline), which are selected on the basis of the mode and route of administration and standard pharmaceutical practice.

Suitable pharmaceutical carriers, as well as pharmaceutical necessities for use in pharmaceutical formulations, are described in Remington's Pharmaceutical Sciences, a standard reference text in this field, and in the USP/NF. A therapeutically effective amount is an amount which is capable of producing a medically desirable result in a treated animal. As is well known in the medical arts, dosage for any one patient depends upon many factors, including the patient's size, body surface area, age, the particular compound to be administered, sex, time and route of administration, general health, and other drugs being administered concurrently. Dosages may vary, but a preferred dosage for intravenous administration of DNA is approximately 10⁶ to 10²² copies of the DNA molecule.

The therapeutic compounds may be administered to a patient by any appropriate method for the particular compound, e.g., orally, intravenously, parenterally, transdermally, transmucosally, by inhalation, or by surgery or implantation at or near the site where the effect of the compound is desired (e.g., with the compound being incorporated into a solid or semi-solid biologically compatible and resorbable matrix). Therapeutic doses are determined specifically for each compound. For non-nucleic acid type compounds, doses are within the range of 0.001 to 100.0 mg/kg body weight or within a range that is clinically determined to be appropriate by one skilled in the art. The compositions of the invention may be administered locally or systemically. Administration will generally be parenterally, e.g., intravenously. As mentioned above, DNA may also be administered directly to the target site, e.g., using a vascular catheter or stent.

### Identification of compounds which inhibit AFABP expression or activity

Compounds that inhibit AFABP expression or activity (thereby inhibiting development of atherosclerosis) are identified by methods ranging from rational drug design to screening of random compounds. The latter method is preferable, as simple and rapid assays for testing such compounds are available. Small organic molecules are desirable candidate compounds for this analysis because such molecules are capable of passing through the plasma membrane to inhibit AFABP production or activity within the cell.

The screening of compounds for the ability to AFABP transcription be carried by identifying compounds that block the binding of trans-acting factors to AFABP promoter sequences. A 5' regulatory region of the AFABP gene is linked to a functional promoter and a reporter gene, e.g., the gene encoding luciferase or alkaline phosphatase, and expression assays in the presence and absence of candidate inhibitory compounds are carried out using known methods. For identification of macrophage-specific inhibitors, the expression assays are carried out in macrophages (or in the presence of macrophage lysates) and the level of expression (in the presence and absence of a candidate compound) compared to the level of expression in adipocytes under the same conditions. For luciferase constructs, the cells harboring the construct are harvested after exposure to the candidate compound and luciferase activity measured; for alkaline phosphatase constructs, the culture medium of the cells is collected and the amount of alkaline phosphatase secreted by the cells into the medium is measured. Promoter constructs containing 5' AFABP enhancer (a 518 base pair sequence contained in the 5' region of the murine AFABP gene at about nucleotides -5.4 kb to -4.9 kb: Table 5) and other regulatory regions (see, e.g., U.S. Pat. No. 5,476,926) are introduced into macrophages and/or adipocytes.

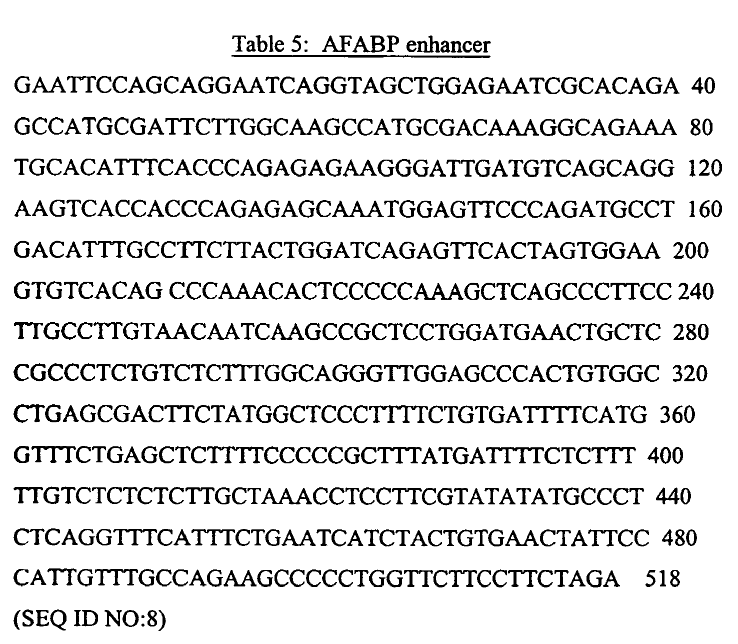

Trans-acting factors which bind to those sequences (or inhibit binding to those sequences) are identified using these constructs. To identify compounds capable of inhibiting AFABP transcription, these cells containing AFABP regulatory sequences are contacted with candidate compounds and the ability the cells to generate the reporter protein is determined (e.g., luciferase in the cells or alkaline phosphatase in the media). A decrease in the amount of expression of the reporter protein indicates that the candidate compound inhibits AFABP transcription.

Candidate compounds may also be screened using cell culture assays. Cells expressing AFABP, e.g., macrophages, adipocytes or epithelial cells, are cultured in the presence of the candidate compound. Any cell regardless of the cell type can be used in the screening assays described herein provided the cells express AFABP. For example, the cells express AFABP from endogenous DNA encoding DNA (either consitutively or induced by an inducing agent) or the cell is genetically-altered to express AFABP, e.g., by introducing into the cell DNA encoding a heterologous AFABP or DNA containing a heterologous promoter operably linked to AFABP coding sequences). The level of expression of AFABP in the presence and absence of the compound is measured using known methods, e.g., PCR or Northern blot analysis to measure transcription. Western blot analysis can be used to detect the presence of the AFABP protein. Alternatively, fatty acid binding, cholesterol loading, or differentiation of a macrophage to a macrophage-derived foam cells is measured to evaluate AFABP activity. To identify compounds capable of inhibiting AFABP activity, cultured macrophages cells or primary cells (e.g., peripheral blood monocytes or macrophages) are contacted with a candidate compound. A control sample of cells is processed in parallel in the absence of a candidate compound. AFABP activity is measured in both samples either by measuring AFABP-fatty acid binding, differentiation of a macrophage into a foam cell, or cholesterol loading in a macrophage. Cholesterol loading by macrophages is determined using known methods, e.g., those described by Yancey et al., 1998, J. Lipid Res. 39:1349-1361. Differentiation of macrophages into foam cells is evaluated histologically, e.g, as described in Tracy, R.E., 1998, Ann. Diagn. Pathol. 2:159□166. RAW 264.7 cells, a murine macrophage cell line, is a suitable model of foam cell formation; alternatively, peripheral blood derived monocytes or macrophages or peritoneal macrophages can be used to study foam cell formation. Binding of fatty acids such as oleic acid and retinoic acid to AFABP is measured using standard reagents, e.g., a fluorescent probe of free fatty acids, e.g., ADIFAB, in standard assay systems such as fluorometry (Richieri et al., 1994, J. Biol. Chem. 269:23918-23930 or Richieri et al., 1996, J. Biol. Chem. 271:11291-11300) or a classical Lipidex 1000 binding assay (Nemecz et al. 1991, Arch. Biochem. Biophys. 286:300-309. A decrease in the amount of fatty acid binding, the level of macrophage differentiation, or cholesterol loading in the presence of the candidate compound compared to that in the absence of the candidate compound indicates that the candidate compound inhibits AFABP activity and thus inhibits atherosclerotic lesion formation.

The screening of compounds for the ability to reduce or block AFABP binding to an intracellular ligand is carried out using in vitro biochemical assays, cell culture assays, or animal model systems. The ligand may be a fatty acid, a synthetic compound (e.g., ETYA), or a peroxisome proliferator□activated receptor (PPAR). Small peptide inhibitors are identified using a commercially-available peptide screening kit (FliTrxJ Random Peptide Display Library, Invitrogen Corporation, Carlsbad, CA). With this screening approach, AFABP is immobilized (e.g., by coated a tissue culture plate or petri dish) and an E. coli bacteriophage expression library plated on the immobilized AFABP. After culturing the plates to allow growth of colonies and expression of the recombinant peptides, the plates are washed. Bacteria which remain bound to the AFABP-coated plate are deemed to express a peptide that binds to AFABP. DNA encoding the binding peptides are identified by PCR (and cloned) according to the manufacturer's instructions. Candidate peptides are then evaluated for the ability to block AFABP binding to fatty acids in cells. Peptides are then tested to determine whether the peptides inhibit AFABP/fatty acid binding preferentially in macrophages (compared to other cells, e.g., adipocytes or epithelial cells). At least 50% more inhibition, preferably at least 75% more inhibition, more preferably at least 100% more inhibition, and most preferably at least 200% more inhibition in one cell type, e.g., a macrophage, compared to another cell type, e.g., an adipocyte, indicates that the peptide preferentially inhibits AFABP/fatty acid binding in a particular cell type. Small peptides identified in this manner are useful as models for designing and producing organic molecules which bind to the same or similar region of AFABP. Peptides which preferentially block AFABP/fatty acid binding in macrophages are incorporated into liposomes which can be targeted to macrophages by coating the liposomes with a composition (e.g., a peptide or polysaccharide) that binds to a macrophage-specific ligand.

In an alternative assay, AFABP is immobilized, e.g., by application to a column, and contacted with a candidate compound and a ligand. For example, the compound is applied to the column before, after, or simultaneously with a labelled ligand (e.g., an intracellular protein or a fatty acid such as oleic acid or retinoic acid labeled with a fluorochrome or a radioisotope), and the amount of labeled ligand bound to AFABP in the presence of the compound is determined by conventional methods. A compound tests positive for inhibiting AFABP binding to a ligand (thereby having the effect of inhibiting AFABP activity or function) if the amount of labeled ligand bound in the presence of the compound is lower than the amount bound in its absence.

Candidate compounds may also be screened using cell-based assays. Cells expressing AFABP, either naturally or after introduction into the cells of genes encoding AFABP or a fragment thereof, are cultured in the presence of the candidate compound. For example, peptides that bind to AFABP (or block binding of AFABP to a ligand thereby inhibiting its in vivo function) are identified using a yeast two-hybrid system and a library of constrained peptides using methods known in the art, e.g., the screening method described in Colas et al., 1996, Nature 380:548-550.

Compounds identified as having the desired effect, i.e., binding to AFABP or inhibiting AFABP binding to its intracellular ligand, are tested further in appropriate animal models, e.g., an animal model of atherosclerosis such as the apoE -/- mice to determine whether the compound reduces lesion formation in vivo.

### Example 1: Decrease of AFABP Expression Prevents the Development of Accelerated Atherosclerosis in Hypercholesterolemic Mice

The following materials and methods were used to evaluate development of atherosclerosis in an art-recognized mouse model of vascular disease.

### Mice

AFABP-/- mice (Hotamisligil et al., 1996, Science 274:1377-1379) were back-crossed and fixed on a C57BL/6J background using known methods (e.g., methods described in Scheja et al., 1999, Diabetes 48:1987-1994). The mice were then bred with ApoE-/- mice (C57BL/6J-Apoe^{tmlUnc}; available from The Jackson Laboratories, Bar Harbor, ME) to generate mice heterozygous for ApoE and AFABP (ApoE+/-AFABP+/-). These mice were then bred to generate mice that were wild-type, deficient in ApoE (ApoE-/-), deficient in AFABP (AFABP-/-), or deficient in both AFABP and ApoE (ApoE-/- AFABP-/-). The mice were genotyped by polymerase chain reaction analysis, which was confirmed by Southern blot analysis. Male mice were weaned at four weeks of age and then placed on a Western diet (Teklad Adjusted Calories Western-type diet, 88137, Harlan-Teklad) containing 21% fat by weight. After 12 weeks on the Western diet the mice were anesthetized and their vasculature was perfused with phosphate buffered saline. Histologic analysis of the vasculature was performed using standard methods.

### In Situ Hybridization

Aortas from wild-type and ApoE-/- mice were perfusion fixed with 4% paraformaldehyde and processed for *in situ* hybridization using standard methods. AFABP mRNA was detected with an antisense ³²P-labeled riboprobe transcribed from a linearized mouse AFABP template. A sense ³²P-labeled riboprobe was used as a negative control.

### Macrophage harvesting, cell culture, and Northern analysis

Peritoneal macrophages were harvested from wild-type C57BL/6J mice. Human peripheral blood monocytes were isolated from buffy coat using a standard Ficoll-Hypaque centrifugation technique. For both mouse and human cells, staining with α-naphthyl butyrate esterase (SIGMA, St. Louis, MO) revealed the cells to be of macrophage origin. 3T3-L1 cells were differentiated to adipocytes in culture using known methods.

Total RNA was obtained from cultured macrophages and 3T3-L1 cells, and

Northern blots were prepared. Filters were hybridized with a ³²P-labeled cDNA probe for mouse AFABP. The human monocyte/macrophage filter was also hybridized with a ³²P-labeled cDNA probe for human scavenger receptor class A (SR-A). To control for differences in RNA loading, the filters were rehybridized with a ³²P-labeled oligonucleotide probe complementary to 28S ribosomal RNA. Immunohistochemical staining and morphometry

The arch of the aorta and the right brachiocephalic artery were dissected out and fixed by immersion in methyl Carnoy's solution prior to embedding in paraffin. Sections of tissue from the same anatomical level of the right brachiocephalic artery were analyzed. Fixed and sectioned vessels from the mice were stained with Verhoeff's stain for elastin to assess lesion areas and luminal occlusion, with Masson trichrome to assess collagen deposition, and with an anti-MOMA-2 antibody to identify macrophages. The areas of the atherosclerotic lesions and the lumen were measured by computerized planimetry, and the percentage of luminal occlusion was calculated as the area of lesion divided by the entire area within the internal elastic lamina, multiplied by 100. To assess collagen and macrophage accumulation, the respective areas of positive staining for Masson trichrome and MOMA-2 (measured by colorimetric analysis) were divided by the entire area of the lesion and multiplied by 100.

### Sudan IV staining of lipid in the aortic arch and its branches

The arch of the aorta and its branches (including the right brachiocephalic artery, the right and left common carotid arteries, and the right and left subclavian arteries) were dissected out, pinned on silicone, and postfixed with formalin. The vasculature was stained with Sudan IV using methods known in the art.

### Lipoprotein analysis

ApoE-/- and ApoE-/- AFABP-/- mice on a Western diet were fasted for four hours in the morning. Blood was then drawn by retro-orbital bleeding into

EDTA-coated tubes. After centrifugation, total cholesterol and triglyceride levels were determined with commercially available enzymatic reaction kits (SIGMA, St. Louis, MO). Cholesterol concentrations in lipoprotein fractions from fasting ApoE-/- and ApoE-/- mice were determined by FPLC (Superose 6 column separation).

### Transplant model of arteriosclerosis

Donor carotid arteries from B10.A-H2^{h2}(2R)/SgSnJ mice (The Jackson Laboratories, Bar Harbor, ME) were transplanted into C57BL/6J recipients that were either wild-type or AFABP deficient. Carotid arteries were harvested 14 days after transplantation and immersion-fixed in methyl Carnoy's solution prior to embedding in paraffin. The allografted carotid arteries were section serially and stained for MOMA-2.

### Effect of AFABP on Development of Atherosclerotic Lesions

Lipid deposition in arterial walls due to elevated levels of plasma cholesterol is central to the development of atherosclerosis. The process is initiated when modified cholesterol, particularly oxidized low-density lipoprotein (oxLDL), is taken up by monocyte-derived macrophages. Peroxisome proliferator activated receptor-γ, AFABP is induced in cultured macrophages by agonists of PPARy. The data described herein demonstrate that atherosclerotic lesions from hypercholesterolemic, apolipoprotein E deficient (ApoE-/-) mice (but not arterial walls from normal mice) contain high levels AFABP mRNA.

AFABP was detected in inflammatory cells that localized in these lesions, as confirmed by its presence in isolated mouse and human macrophages. To determine the importance of AFABP in atherosclerosis, mice were lacking both ApoE and AFABP (ApoE-/-AFABP-/-) were made. In comparison with ApoE-/- mice, ApoE-/- AFABP-/- mice developed trivial lesions that were markedly smaller, less complex, and less microphage-rich even though the ApoE-/-AFABP-/- mice remained hypercholesterolemic. Conversely, absence of AFABP did not prevent lesion formation and macrophage accumulation in transplant-associated arteriosclerosis that does not depend on elevated levels of cholesterol. These results indicate that AFABP participates in the development of hypercholesterolemia-induced atherosclerosis.

### AFABP is present in the atherosclerotic lesions

The development of an atherosclerotic lesion is initiated by injury to the arterial wall. In individuals with hypercholesterolemia, LDL and its modified forms are a critical source of injury leading to an inflammatory response. Circulating monocytes enter the arterial wall, differentiate into macrophages, and then scavenge cholesteryl esters that originate from plasma lipoproteins (particularly oxLDL) to become lipid-filled foam cells. When these foam cells accumulate within the intima (inner layer of the artery), the first identifiable lesion of arteriosclerosis, the fatty-streak, develops. As the atherosclerotic lesion progresses, migration and proliferation of smooth muscle cells and deposition of fibrous tissue lead to an advanced, complicated lesion. To investigate whether AFABP is important in development of atherosclerosis, *in situ* hybridization was carried out to determine if AFABP was expressed in atherosclerotic lesions. AFABP mRNA was not detectable in the arterial walls of wild-type mice except in adipose tissue of the adventitia-outer surrounding connective tissue of the artery-by *in situ* hybridization with an antisense probe. However, intense

AFABP signal was visible in the arterial walls of ApoE-/- mice. The signal for AFABP was prominent in both the atherosclerotic lesion and the adipose tissue of the adventitia, with much less signal in the media (middle, muscular portion of the arterial wall). In addition, AFABP and mRNA was not restricted to areas of accumulated lipid in the lesion of the adventitia. No signal was detectable in wild-type or ApoE-/- vessels when a sense AFABP probe was used as a negative control.

### AFABP mRNA is induced in mouse and human macrophages

To determine which cells in atherosclerotic lesions other than adipocytes express AFABP, the *in situ* hybridization signal for AFABP was compared to the level of immunostaining for MOMA-2 (a macrophage-specific marker) in lesions from ApoE-/- mice. Areas of AFABP signal overlapped areas of macrophage staining in these lesions, indicating that macrophages express AFABP. Peritoneal macrophages were harvested from wild-type mice, and the level of AFABP mRNA was evaluated. As a positive control, AFABP mRNA levels were measured in 3T3-L1 cells that had been differentiated into adipocytes. AFABP mRNA was present in mouse peritoneal macrophages, and the level of AFABP message increased one day after the cells had been plated on plastic (a stimulus for macrophage differentiation). AFABP message increased when the macrophages were exposed to oxLDL but not when they were exposed to non-oxidized LDL. AFABP mRNA levels during the differentiation of human monocytes to macrophages was also measured. AFABP message was found not to be expressed during early differentiation, but it became detectable three days after the cells had been plated on plastic. The level of AFABP mRNA was highest after 5 days. This pattern of AFABP mRNA induction in mature human macrophages paralleled the increase in scavenger receptor class A (SR-A) mRNA, a marker of monocyte to macrophage differentiation and a receptor important for oxLDL uptake during atherogenesis. These data demonstrate that AFABP is expressed in both mouse and human macrophages and that AFABP is induced by oxLDL, a stimulus for the development of atherosclerosis.

The level of AFABP mRNA was examined in vascular smooth muscle cells, another cell type predominant in atherosclerotic lesions. Compared to the level detected in macrophages and adipocytes, AFABP mRNA levels in vascular smooth muscle cells from adult mice was very greatly reduced.

### Lipoprotein analysis in ApoE -/- and ApoE-/-AFABP-/- mice

AFABP has been reported to play a role in intracellular fatty acid transport, obesity-associated insulin resistance, and cellular lipid metabolism. As described above, mice deficient in both ApoE and AFABP were generated. ApoE-/- mouse model was chosen because ApoE-/- mice develop severe hypercholesterolemia and atherosclerotic lesions characteristic of human disease.

An initial characterization of the ApoE-/-AFABP-/- mice revealed no differences in food intake or body weight (34.6±1.2g, n=17) compared with ApoE-/- mice (36.7±2.2g, n=12) after 12 weeks on a high-fat "Western" diet. Evaluation of serum lipid profiles showed an overall reduction (P<0.05) in total circulating cholesterol levels (Fig. 3A) in ApoE-/-AFABP-/- mice (735±58 mg/dl, n=15) as compared with ApoE-/- mice (1192±137 mg/dl, n=10). However, total cholesterol levels do not exceed 150 mg/dl on western diet. Total circulating triglyceride levels did not differ in ApoE-/-AFABP-/- and ApoE-/- mice (Fig. 3B).

Distribution of cholesterol was characterized in various lipoprotein fractions from the two groups by fast phase liquid chromatography (FPLC). Unlike the plasma of wild-type mice in which high density lipoprotein (HDL) predominates as the major cholesterol-carrying lipoprotein, the plasma of ApoE-/-AFABP-/- and

ApoE-/- mice both showed a predominance of lower density lipoproteins (Fig. 3C-D, mean of 3 in each group). The FPLC patterns for lipoprotein fractions were similar in ApoE-/-AFABP-/- and ApoE-/- mice.

### Absence of AFABP of in ApoE-/- mice prevents fat accumulation and lesion formation in arteries

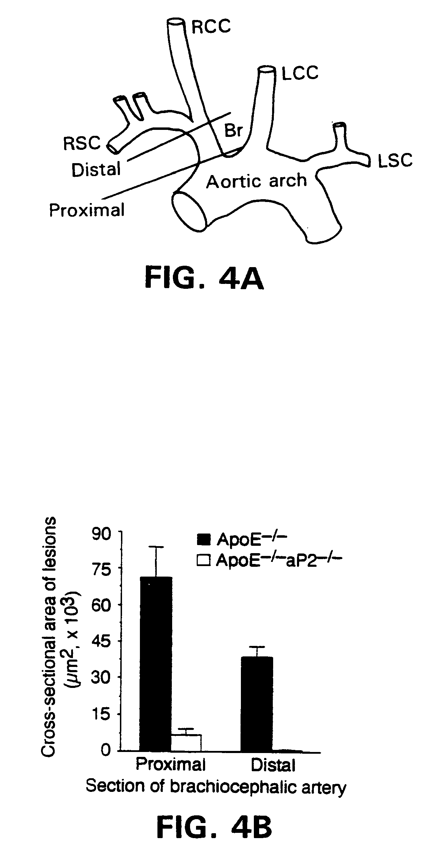

Atherosclerotic lesion formation was examined in the aortic arch and its branches in animals from the two groups. Sudan IV staining revealed a marked decrease in lipid accumulation in ApoE-/-AFABP-/- mice compared to ApoE-/- mice. To characterize the magnitude of the lesions and the severity of vessel obstruction, the cross-sectional area of lesions was measured in the proximal and distal portions of the right brachiocephalic artery (Figs. 4A-B). Large occlusive lesions were present in the proximal (71 ± 12x10³ µm², n=7) and distal (39±4x10³ µm², n=7) brachiocephalic arteries of ApoE-/- mice. These lesions occluded 80±5% of the proximal, and 55±7% of the distal arteries. The size of the atherosclerotic lesion in the ApoE-/- mouse with the lowest serum cholesterol was not different from the mouse with the highest serum cholesterol, suggesting no direct correlation between cholesterol levels and lesion size in these mice. In contrast to the ApoE-/- mice, lesions in ApoE-/-AFABP-/- mice were small and non-occlusive in both the proximal (7±2x10³ µm², n=12) and the distal (0.5±0.3x10³ µm², n=12) brachiocephalic arteries. Five out of 12 ApoE-/-AFABP-/- mice did not develop any detectable atherosclerotic lesions after 12 weeks on a Western diet. Even after 25 weeks on a Western diet, ApoE-/-AFABP-/- mice had small, non-occlusive lesions. Neither wild-type mice nor AFABP-/- mice developed atherosclerosis in the brachiocephalic arteries on Western diet.

### Lesions from ApoE-/- AFABP-/- mice are less advanced and contain fewer macrophages than do those from ApoE-/- mice

Proximal brachiocephalic arteries from representative ApoE-/- and ApoE-/-

AFABP-/- mice were subjected to immunohistochemical analysis. Tissue was stained for elastin, collagen, and MOMA-2, a marker of macrophages. MOMA-2 staining for macrophages was also performed in carotid arteries transplanted into wild-type and

AFABP-/- mice. Atherosclerotic lesions from ApoE-/- mice were complex lesions with fibrous caps, and the lesions contained large amounts of collagen. The clear areas within these advanced, complicated lesions are areas of lipid accumulation. ApoE-/-AFABP-/- mice, in contrast, had very small, non-complicated lesions that laced fibrous caps and deposition of excess extracellular matrix. In ApoE-/- mice, 45±5% (n=4) of the lesions was composed of collagen; while only 13±4% (n=4) of the lesion was composed of collagen in ApoE-/-

AFABP-/- mice. Macrophage accumulation in the two groups was assessed by immunostaining the arteries for MOMA-2. The percentage of lesions staining positive for MOMA-2 in the proximal arteries was thirty-fold higher in ApoE-/- mice than in ApoE-/-AFABP-/- mice.

To determine if AFABP deficiency prevented lesion formation in another type of occlusive vascular disease, lesion size and macrophage accumulation was evaluated in an art-recognized model of transplant-associated arteriosclerosis that does not depend on hypercholesterolemia. No difference in lesion size and no decrease in macrophage accumulation was detected in donor carotid arteries from mice of a different genetic background transplanted into wild-type and AFABP-/- mice. In fact, the lesion in the AFABP-/- mouse contained more macrophages. These data indicate that the lack of lesion formation and macrophage accumulation in the absence of AFABP is specific to hypercholesterolemia-induced atherosclerosis; it was not observed in immune-induced arteriosclerosis.

The results indicate that AFABP is an important mediator of hypercholesterolemia-induced atherosclerosis. It is unlikely that the modest reduction in plasma cholesterol levels in ApoE-/-AFABP-/- mice accounts for the decrease in atherosclerotic lesions in this group, as plasma cholesterol levels remained markedly high in this and the ApoE-/- group. This is supported by the observation that hypolipidemic drugs that decrease plasma cholesterol levels by 30 to 39% in ApoE-/- mice (an amount analogous to the decrease in our ApoE-/- AFABP-/- mice) do not reduce atherosclerotic lesion formation. When the response of macrophages to oxLDL was evaluated, no difference in oxLDL uptake, cholesterol esterification, or foam cell formation in cells from ApoE-/- AFABP-/- and AFABP-/- mice was observed. However, in ApoE-/-AFABP-/- mice, there was a marked decrease in lesion macrophage accumulation. The macrophage response occurred only in the setting of hypercholesterolemia. Macrophage accumulation in transplanted arteries, or in the intraperitoneal cavity after thioglycolate administration was not different in the presence or absence of AFABP. These data indicate that AFABP is an important mediator in the development of atherosclerosis induced by hypercholesterolemia, and that a lack of AFABP leads to a significant decrease in macrophage accumulation and complex-lesion formation.

Other embodiments are within the following claims.

## Claims

1. Use of a compound that reduces expression of AFABP in the manufacture of a medicament for inhibiting formation of an atherosclerotic lesion, wherein said compound is an antisense nucleic acid.

2. The use of claim 1, wherein said compound inhibits transcription of said AFABP.

3. The use of claim 1 or 2, wherein said compound inhibits expression of said AFABP in macrophages but not in adipocytes.

4. The use of claim 1 or 2, wherein said 35 compound inhibits expression of said AFABP in adipocytes but not in macrophages.

5. The use of any one of claim 1 or 2, wherein said compound inhibits expression of said AFABP in both macrophages and adipocytes.

6. The use of claim 1, wherein said antisense nucleic acid molecule comprises at least 10 nucleotides the sequence of which is complementary to an mRNA encoding an AFABP polypeptide.

7. The use of claim 7, wherein said antisense nucleic acid is a DNA operatively linked to a macrophage-specific promoter, wherein transcription of said DNA yields nucleic acid product which is complementary to an mRNA encoding an AFABP polypeptide.

8. The use of claim 1 wherein said compound inhibits differentiation of a macrophage into a foam cell.

9. An *in vitro* method for identifying a compound which inhibits development of an atherosclerotic lesion, comprising the steps of:
(a) contacting AFABP with a fatty acid in the presence of a candidate compound; and
(b) determining the level of AFABP binding to said fatty acid, wherein a decrease in said level of binding in the presence of said candidate compound, compared to the level of binding in the absence of said candidate compound indicates that said compound inhibits development of an atherosclerotic lesion.

10. The method of claim 9 wherein said AFABP and said fatty acid are bound to form a complex.

11. An *in vitro* method for identifying a compound which inhibits AFABP expression in a cell, said method comprising the steps of:
(a) providing a macrophage that expresses AFABP;
(b) culturing said macrophage in the presence of a candidate compound; and
(c) determining the level of expression of a AFABP in said macrophage, wherein an increase in said level of expression in the presence of said candidate compound compared to the level of expression in the absence of said candidate compound indicates that said candidate compound inhibits AFABP expression in said macrophage.

12. An *in vitro* method for determining the ability of a candidate compound to inhibit binding of AFABP to an intracellular ligand in a macrophage, said method comprising the steps of:
(a) providing a macrophage that expresses AFABP;
(b) culturing said macrophage in the presence of a candidate compound; and
(c) determining the level of fatty acid binding in said macrophage, wherein a decrease in said level of binding in the presence of said compound, compared to the level of binding in the absence of said compound, is an indication of the ability of said candidate compound to inhibit AFABP/fatty acid binding in a macrophage.

## Patentansprüche

1. Verwendung einer Verbindung, die die Expression von AFABP verringert, bei der Herstellung eines Arzneimittels zur Hemmung der Bildung einer atherosklerotischen Verletzung, wobei die Verbindung eine Antisense-Nucleinsäure ist.

2. Verwendung nach Anspruch 1, bei der die Verbindung die Transkription des AFABP hemmt.

3. Verwendung nach Anspruch 1 oder 2, bei der die Verbindung die Expression des AFABP in Makrophagen, aber nicht in Adipozyten hemmt.

4. Verwendung nach Anspruch 1 oder 2, bei der die Verbindung die Expression des AFABP in Adipozyten, aber nicht in Makrophagen hemmt.

5. Verwendung nach einem der Ansprüche 1 oder 2, bei der die Verbindung die Expression des AFABP sowohl in Makrophagen als auch in Adipozyten hemmt.

6. Verwendung nach Anspruch 1, bei der das Antisense-Nucleinsäuremolekül mindestens 10 Nucleotide aufweist, deren Sequenz komplementär zu einer mRNA ist, die ein AFABP-Polypeptid codiert.

7. Verwendung nach Anspruch 6, bei der die Antisense-Nucleinsäure eine DNA ist, die funktionsfähig an einen Makrophagen-spezifischen Promotor gebunden ist, wobei die Transkription der DNA ein Nucleinsäure-Produkt ergibt, das komplementär ist zu einer mRNA, die ein AFABP-Polypeptid codiert.

8. Verwendung nach Anspruch 1, bei der die Verbindung die Differenzierung eines Makrophagen zu einer Schaumzelle hemmt.

9. In vitro-Verfahren zum Identifizieren einer Verbindung, die die Entwicklung einer atherosklerotischen Verletzung hemmt, folgende Schritte aufweisend.
(a) in-Berührung-Bringen von AFABP mit einer Fettsäure in Anwesenheit einer Prüfverbindung; und
(b) Bestimmen des Ausmaßes des AFABP-Bindung an die Fettsäure, wobei ein Sinken des Ausmaßes der Bindung in Anwesenheit der Profverbindung, verglichen mit dem Ausmaß der Bindung in Abwesenheit der Prüfverbindung, anzeigt, dass die Verbindung die Entwicklung einer atherosklerotischen Verletzung hemmt.

10. Verfahren nach Anspruch 9, bei dem das AFABP und die Fettsäure gebunden sind, um einen Komplex zu bilden.

11. In vitro-Verfahren zum Identifizieren einer Verbindung, die die AFABP-Expression in einer Zelle hemmt, wobei das Verfahren folgende Schritte aufweist:
(a) Bereitstellen eines Makrophagen, derAFABP exprimiert;
(b) Kultivieren des Makrophagen in Anwesenheit einer Prüfverbindung; und
(c) Bestimmen des Ausmaßes der Expression eines AFABP in dem Makrophagen, wobei ein Sinken des Ausmaßes der Expression in Anwesenheit der Prüfverbindung, verglichen mit dem Ausmaß der Expression in Abwesenheit der Prüfverbindung, anzeigt, dass die Prüfverbindung die AFABP-Expression in dem Makrophagen hemmt.

12. In vitro-Verfahren zur Bestimmung der Fähigkeit einer Prüfverbindung, die Bindung von AFABP an einen intrazellulären Liganden in einem Makrophagen zu hemmen, wobei das Verfahren folgende Schritte aufweist:
(a) Bereitstellen eines Makrophagen, der AFABP exprimiert;
(b) Kultivieren des Makrophagen in Anwesenheit einer Prüfverbindung; und
(c) Bestimmen des Ausmaßes der Fettsäure-Bindung in dem Makrophagen, wobei ein Sinken des Ausmaßes der Bindung in Anwesenheit der Verbindung, verglichen mit dem Ausmaß der Bindung in Abwesenheit der Verbindung, ein Hinweis auf die Fähigkeit der Prüfverbindung, die AFABP/Fettsäure-Bindung in einem Makrophagen zu hemmen, ist.

## Revendications

1. Utilisation d'un composé qui réduit l'expression d'une AFABP dans la fabrication d'un médicament pour inhiber la formation d'une lésion athérosclérotique, dans laquelle ledit composé est un acide nucléique antisens.

2. Utilisation selon la revendication 1, dans laquelle ledit composé inhibe la transcription de ladite AFABP.

3. Utilisation selon la revendication 1 ou 2, dans laquelle le composé inhibe l'expression de ladite AFABP dans des macrophages mais pas dans des adipocytes.

4. Utilisation selon la revendication 1 ou 2, dans laquelle ledit composé (35) inhibe l'expression de ladite AFABP dans des adipocytes mais pas dans des macrophages.

5. Utilisation selon l'une quelconque des revendications 1 et 2, dans laquelle ledit composé inhibe l'expression de ladite AFABP à la fois dans des macrophages et dans des adipocytes.

6. Utilisation selon la revendication 1, dans laquelle ladite molécule d'acide nucléique antisens comprend au moins 10 nucléotides dont la séquence est complémentaire d'un ARNm codant un polypeptide d'AFABP.

7. Utilisation selon la revendication 7, dans laquelle ledit acide nucléique antisens est un ADN lié de manière opérationnelle à un promoteur spécifique d'un macrophage, dans laquelle la transcription dudit ADN donne un produit d'acide nucléique qui est complémentaire d'un ARNm codant un polypeptide d'AFABP.

8. Utilisation selon la revendication 1, dans laquelle ledit composé inhibe la différentiation d'un macrophage en une cellule spumeuse.

9. Procédé *in vitro* pour identifier un composé qui inhibe le développement d'une lésion athérosclérotique, comprenant les étapes consistant à :
(a) mettre en contact de l'AFABP avec un acide gras en présence d'un composé candidat ; et
(b) déterminer le niveau de liaison de l'AFABP audit acide gras, une diminution dudit niveau de liaison en présence dudit composé candidat, en comparaison avec le niveau de liaison en l'absence dudit composé candidat, indiquant que ledit composé inhibe le développement d'une lésion athérosclérotique.

10. Procédé selon la revendication 9, dans lequel ladite AFABP et ledit acide gras sont liés pour former un complexe.

11. Procédé *in vitro* pour identifier un composé qui inhibe l'expression d'une AFABP dans une cellule, ledit procédé comprenant les étapes consistant à :
(a) se procurer un macrophage qui exprime une AFABP ;
(b) cultiver ledit macrophage en présence d'un composé candidat ; et
(c) déterminer le niveau d'expression d'une AFABP dans ledit macrophage, une augmentation dudit niveau d'expression en présence dudit composé candidat, en comparaison avec le niveau d'expression en l'absence dudit composé candidat indiquant que ledit composé candidat inhibe l'expression de l'AFABP dans ledit macrophage.

12. Procédé *in vitro* pour déterminer l'aptitude d'un composé candidat à inhiber la liaison d'une AFABP à un ligand intracellulaire dans un macrophage, ledit procédé comprenant les étapes consistant à :
(a) se procurer un macrophage qui exprime une AFABP ;
(b) cultiver ledit macrophage en présence d'un composé candidat ; et
(c) déterminer le niveau de liaison à un acide gras dans ledit macrophage, une diminution dudit niveau de liaison en présence dudit composé, en comparaison avec le niveau de liaison en l'absence dudit composé, étant une indication de l'aptitude dudit composé candidat à inhiber la liaison AFABP/acide gras dans un macrophage.
